# EUROPÄISCHE PATENTSCHRIFT

(11) **EP 3 436 699 B1**
(45) Veröffentlichungstag und Bekanntmachung des Hinweises auf die Patenterteilung: **28.10.2020**
(21) Anmeldenummer: 17713659.5
(22) Anmeldetag: 27.03.2017
(51) Int. Cl.: F03G 7/06, H02N 2/02

(54) **ELEKTRISCHER LINEARER REPETITIVER IMPULSANTRIEB ZUM BETRIEB VON GERÄTEN**
ELECTRIC LINEAR REPETITIVE PULSED DRIVE FOR OPERATING EQUIPMENT
MOTEUR À IMPULSION RÉPÉTITIF LINÉAIRE ÉLECTRIQUE POUR FAIRE FONCTIONNER DES APPAREILS

(30) Priorität: 27.03.2016 DE 102016003599
(43) Veröffentlichungstag der Anmeldung: 06.02.2019
(73) Patentinhaber: Eto Magnetic GmbH, 78333 Stockach (DE)
(72) Erfinder: BLANK, Anton, 70173 Stuttgart (DE)
(74) Vertreter: Daub, Thomas
(86) Internationale Anmeldenummer: PCT/EP2017/057181
(87) Internationale Veröffentlichungsnummer: WO 2017/167684

(56) Entgegenhaltungen:
- EP-A1- 3 631 872
- WO-A1-2014/019738
- WO-A1-2016/038028
- WO-A1-2016/046142
- DE-A1-102005 038 891
- DE-A1-102013 107 744
- JP-A- H04 253 888
- JP-A- H04 253 889
- JP-A- 2004 162 703
- US-A1- 2010 242 673
- US-B1- 6 515 382
- FENG-XIANG WANG ET AL: "Design and control of linear actuators made by magnetically controlled shape memory alloy", MECHATRONICS, 2005. ICM '05. IEEE INTERNATIONAL CONFERENCE ON TAIPEI, TAIWAN 10-12 JULY 2005, PISCATAWAY, NJ, USA,IEEE, US, 10 July 2005 (2005-07-10), pages 583-586, XP010848042, DOI: 10.1109/ICMECH.2005.1529323 ISBN: 978-0-7803-8998-4
- DATABASE INSPEC [Online] THE INSTITUTION OF ELECTRICAL ENGINEERS, STEVENAGE, GB; 28 August 2000 (2000-08-28), ULLAKKO K ET AL: "Magnetic shape memory (MSM)-a new way to generate motion in electromechanical devices", Database accession no. 7355113 & PROCEEDINGS OF INTERNATIONAL CONFERENCE ON ELECTRICAL MACHINES, vol. 3, 28 August 2000 (2000-08-28), page 1195, ICEM 2000 Proceedings. International Conference on Electrical Machines Helsinki Univ. Technol. Espoo, Finland ISBN: 951-22-5097-7
- QINGXIN ZHANG ET AL: "A Novel Linear Actuator and Its Controlling System", AUTOMATION AND LOGISTICS, 2007 IEEE INTERNATIONAL CONFERENCE ON, IEEE, PISCATAWAY, NJ, USA, 1 August 2007 (2007-08-01), pages 979-984, XP031138916, ISBN: 978-1-4244-1531-1
- FENG-XIANG WANG ET AL: "Actuation principle and property of magnetically controlled shape memory alloy actuators", MECHATRONICS, 2005. ICM '05. IEEE INTERNATIONAL CONFERENCE ON TAIPEI, TAIWAN 10-12 JULY 2005, PISCATAWAY, NJ, USA,IEEE, US, 10 July 2005 (2005-07-10), pages 579-582, XP010848041, DOI: 10.1109/ICMECH.2005.1529322 ISBN: 978-0-7803-8998-4

## Beschreibung

Die Erfindung betrifft einen elektrischen linearen repetitiven Impulsantrieb zum Betrieb von Geräten, für deren Wirkungsverbesserung eine impulsartig abgegebene Arbeitsenergie bei gleichzeitig niedrigen Wiederholfrequenzen von Vorteil ist. Zur Verdeutlichung elektrischer Geräte, die durch diese Patentanmeldung verbessert werden können, seien beispielhaft genannt: Geräte, die in organische Haut einstechen oder die Stoffe in organische Haut einstechen, also beispielsweise für Permanent Make-up, Tätowierungen, Microblading, Mesotherapie, Micro-Needling, Reiztherapie, Narbenbehandlung, Einbringen von körpereigenen Stoffen, wie Hyaluronsäure, Collagen etc. bei welchen die Elastizität der Haut umso besser überwunden werden kann, je höher die Einstechgeschwindigkeit ist und die Empfindlichkeit der Haut in Bezug auf eine mechanische Behandlung nur geringe Wiederholfrequenzen zulässt, weil sonst zu große Wunden und nachfolgende Vernarbungen auftreten können und weiterhin beispielhaft, Rasierapparate, Haarschneider, Epiliergeräte bei denen eine hohe Schnitt- oder Rupfgeschwindigkeit für eine schmerzarme Anwendung sorgt, die mit Apparaten des Stands der Technik, wie auch im vorangegangenen Beispiel, nur mit hohen Wiederholfrequenzen erreichbar wäre und damit zu rascher Erwärmung, starken Vibrationen, großer Geräuschentwicklung und, soweit es sich um akku-/batteriebetriebene Geräte handelt, durch den großen Leistungsbedarf auch zum raschen Erschöpfen des Akkus/der Batterie führen würden und weiterhin beispielhaft, Zahnreinigungsgeräte, Manikürgeräte, Pedikürgeräte weil beispielsweise die Arbeitsgeschwindigkeit des Antriebs zu wesentlich kürzeren Behandlungszeiten und die geringe Wiederholfrequenz zu einer schonenderen Behandlung führt.

### Stand der Technik

Elektrische lineare, also nicht rotierende, sondern auf einer Bewegungsachse repetitive Antriebe basieren üblicherweise auf linearen elektromagnetischen Zug/Druckmagneten oder auf rotierenden Motoren, deren Rotation über ein Getriebe in eine linearen Bewegung umgesetzt wird. Weitere Wirkprinzipien, wie das piezoelektrische, das magnetostriktive oder das elektrostatische Prinzip erreichen zwar die gewünschten hohen Geschwindigkeiten von <1 ms (1/1000 Sekunde) pro Arbeitshub, doch ist deren Arbeitsweg für die Anwendungen zu klein oder deren Preis für eine Massenproduktion zu hoch. Das thermische Wirkprinzip und der Formgedächtniseffekt erbringen umgekehrt die gewünschten größeren Wege von >0,5 mm, jedoch mit viel zu geringer Arbeitsgeschwindigkeit.

Die heute vorwiegend verwendeten Antriebe zur elektrischen Erzeugung linearer repetitiver Bewegungen, die elektromagnetischen Zug/Druckmagnete, oszillieren aufgrund ihres Aufbaus als Masse- Feder-System oder als rotierende elektrische Motoren mit Getriebe zeitkontinuierlich entsprechend einem näherungsweise sinusförmigen Weg-ZeitDiagramm. Damit sind alle Bewegungsparameter ebenfalls zeitkontinuierlich näherungsweise sinusförmig über die Zeit verteilt, so dass eine Konzentration der eingebrachten Energie auf einen kurzen Zeitraum mit hoher Geschwindigkeit nicht möglich ist, also kurze schnelle zeitdiskontinuierliche Bewegungen prinzipbedingt nicht erzielt werden können, außer man erhöht die Wiederholfrequenz, die bei den genannten Anwendungen zu gravierenden Nachteilen führen würden, wie Erwärmung, Geräuschentwicklung, Vibrationen.

Die einzigen elektrischen linearen repetitiven Antriebe, welche den Anforderungen nach Hub und Geschwindigkeit bei massenproduktionstauglichen Preisen gerecht werden, sind die elektrischen Klappanker- respektive Flachankermagnete. Bei beiden ähnlichen Funktionsweisen, wird durch Anzug eines ferromagnetischen Ankerteils ein magnetischer Kreis geschlossen, so dass der Anker bis zum Aufschlagen auf den Stator überproportional beschleunigt wird und auf diese Weise ausreichend hohe Geschwindigkeiten und Hübe erreicht werden. Das abrupte Abbremsen des Ankers beim Aufschlagen auf den Stator vernichtet in sehr kurzer Zeit einen Großteil der elektrisch eingebrachten Bewegungsenergie und erzeugt dadurch entsprechend laute Geräusche, die für die genannten praktischen Anwendungen nicht hinnehmbar sind. Dämpft man das Aufschlagen des Ankers, beispielsweise durch elastische Materialien oder Federn, so gehen die Vorteile dieses Prinzips verloren, da aufgrund der kleinen Hübe von ca. 0,5-1,5 mm auch nur kleine Bremswege zur Verfügung stehen und dadurch die höchstmögliche Geschwindigkeit auf Kosten einer geringeren Geräuschentwicklung drastisch reduziert wird.

Die bekannten und angewendeten Wirkprinzipien elektrischer linearer repetitiver Antriebe mit zeitkontinuierlicher Energieabgabe führen zu begrenzten Geschwindigkeiten, eingeschränkten Hüben oder inakzeptabler Geräuschentwicklung und somit zu eingeschränkter Leistungsfähigkeit der mit diesen Wirkprinzipien betriebenen Geräte.

Dokumente FENG-XIANG WANG ET AL: "Design and control of linear actuators made by magnetically controlled shape memory alloy", MECHATRONICS, 2005. ICM '05. IEEE INTERNATIONAL CONFERENCE ON TAIPEI, TAIWAN 10-12 JULY 2005, PISCATAWAY, NJ, USA,IEEE, US, 10. Juli 2005 (2005-07-10), Seiten 583-586, XP010848042, DOI: 10.1109/ ICMECH.2005.1529323 ISBN: 978-0-7803-8998-4, US 2010/242673 A1 und WO 2016/038028 A1 offenbaren MSMA-basierte Aktuatoren.

### Vorteile der Erfindung

Aufgabe der Erfindung ist es, einen elektrischen linearen repetitiven Antrieb zu schaffen, der eine impulsartige zeitdiskontinuierliche Energieabgabe mit höheren Arbeitsgeschwindigkeiten bei mindestens gleichem Hub, jedoch bei wesentlich kleineren Wiederholfrequenzen als die Antriebe des Stands der Technik bereitstellt, ohne dass Geräuschentwicklung, Vibrationen oder Erwärmung die gewünschten Anwendungsfunktionen einschränken. Ferner liegt der Erfindung insbesondere die Aufgabe zugrunde, einen Antrieb insbesondere für Körperpflegegeräte und/oder Körperbehandlungsgeräte wie beispielsweise Rasierapparate, Haarschneider, Bartschneider, Zahnbürsten oder dergleichen mit vorteilhaften Eigenschaften hinsichtlich einer erzeugbaren Bewegung und/oder einer Kraftentfaltung bereitzustellen.

Diese Aufgabe wird erfindungsgemäß mit einer Vorrichtung nach dem unabhängigen Anspruch 1 dadurch gelöst, dass der elektrische lineare repetitive Impulsantrieb durch ein im Luftspalt eines magnetischen Kreises befindliches magnetisches Formgedächtnis-Stellelement gebildet ist, dieses Stellelement durch einen Stromimpuls in der Spule des magnetischen Kreises zu einer schnellen Dehnung veranlasst wird und dass die magnetische Energie, die durch diesen Stromimpuls im ferromagnetischen Material des magnetischen Kreises gespeichert wurde, im Umkehrpunkt des Arbeitsstromimpulses durch einen gegensinnigen Stromimpuls gelöscht wird, um das Stellelement mittels einer Rückstelleinheit, insbesondere einer Rückstellfeder, mit maximaler Schnelligkeit in die Ausgangslage zurück zu bringen. Ferner wird die Aufgabe insbesondere durch einen elektrischen linearen repetitiven Impulsantrieb gemäß dem Anspruch 1 gelöst. Weiterhin sind vorteilhafte Weiterbildungen und Ausgestaltungen der Erfindung den weiteren Ansprüchen zu entnehmen.

Ein wesentlicher Vorteil, welcher gegenüber dem Stand der Technik erreicht wird, besteht darin, dass der erfindungsgemäße elektrische lineare repetitive Impulsantrieb seine Energie zeitdiskontinuierlich, in kurzen Zeitabschnitten, also in Impulsen, gebündelt abgibt und damit vorteilhaft für jeden einzelnen Impuls die maximale Systemenergie abgegeben werden kann und besonders vorteilhaft gleichzeitig Wiederholfrequenzen bis herab zu manuellen Einzelimpulsauslösungen ermöglicht werden.

Vorteilhaft weist der elektrisch lineare repetitive Impulsantrieb einen Anschlag, insbesondere für das Formgedächtnis-Stellelement auf. Besonders bevorzugt ist der Anschlag unmagnetisch. Es ist denkbar, dass die Rückstelleinheit die Rückstellfeder umfasst. Ferner ist denkbar, dass die Rückstelleinheit als die Rückstellfeder ausgebildet ist.

Ein weiterer wesentlicher Vorteil, welcher gegenüber dem Stand der Technik erreicht wird, besteht darin, dass jeder der Parameter, Anstiegszeit, Anstiegsverlauf, Auslenkung, Impulsdauer, Abfallzeit, Abfallverlauf und Wiederholfrequenz werksseitig auf den jeweiligen Anwendungsfall oder manuell vom Anwender zur Optimierung der jeweiligen, auch individuellen Anwendung eingestellt werden kann und beim Verändern von ausschließlich der Wiederholfrequenz, die abgegebene Energie pro Arbeitsimpuls konstant bleibt. Insbesondere kann eine pro Arbeitsimpuls abgegebene Energie bei einem Verändern einer Wiederholfrequenz konstant bleiben. Ferner ist denkbar, dass eine Anstiegszeit und/oder ein Anstiegsverlauf und/oder eine Auslenkung und/oder eine Impulsdauer und/oder eine Abfallzeit und/oder ein Abfallverlauf und/oder die Wiederholfrequenz einstellbar sind.

Ein weiterer bedeutender Vorteil der Ausgestaltung der Erfindung wird erfindungsgemäß dadurch erreicht, dass insbesondere zur Minimierung der Rückstellzeit des Impulsantriebs ein umgekehrt gepolter Stromimpuls, also vorteilhaft ein Löschimpuls, welcher bevorzugt im Umkehrpunkt des Arbeitsimpulses ausgelöst wird, das Magnetfeld des magnetischen Kreises schlagartig löscht und dadurch das Stellelement vorteilhaft ungehindert und vorzugsweise mit maximal möglicher Schnelligkeit durch die Rückstellfeder in seine Ausgangslänge zurückgebracht wird.

Ein weiterer bedeutender Vorteil der Ausgestaltung der Erfindung wird erfindungsgemäß dadurch erreicht, dass zur Minimierung der Anstiegszeit des Impulsantriebs die Rückstelleinheit ein weiteres gesteuertes magnetisches Formgedächtnis-Stellelement umfasst und damit die Anstiegszeit durch Entfall der hemmenden Wirkung der Rückstellfeder weiter verbessert wird. Vorteilhaft ist insbesondere in diesem Fall die Rückstelleinheit frei von einer Rückstellfeder. Insbesondere ersetzt in diesem Fall das weitere gesteuerte magnetische Formgedächtnis-Stellelement die Rückstellfeder.

Ein weiterer wesentlicher Vorteil der Erfindung gegenüber dem Stand der Technik besteht darin, dass die schon inhärent moderate Geräuschentwicklung des Festkörper-Stellelements (5) des Impulsantriebs durch geräuschoptimierende Anstiegs- und Abfallkurvenverläufe des Magnetfelds weiter verbessert werden kann und deswegen auf eine leistungsmindernde akustische Dämpfung der Kinematik und des den Impulsantrieb umgebenden Gehäuses verzichtet werden kann. Insbesondere ist der elektrische lineare repetitive Impulsantrieb frei von einer Dämpfung. Ferner ist der elektrische lineare repetitive Impulsantrieb insbesondere frei von einem Gehäuse.

Ein weiterer Vorteil der Erfindung gegenüber dem Stand der Technik besteht darin, dass sich aufgrund der zeitdiskontinuierlichen Impulsansteuerung des Impulsantriebs gegenüber Antrieben des Stands der Technik wesentlich geringere Störgeräusche, kleinere Vibrationen, eine geringere Erwärmung und eine längere Betriebsdauer mit Akkus/Batterien beim Betrieb der genannten Geräte ergeben.

Der elektrische lineare repetitive Impulsantrieb entfaltet die beschriebenen Vorteile in Geräten, für deren Wirkungsverbesserung eine kurze impulsartig abgegebene Arbeitsenergie bei gleichzeitig niedrigen Wiederholfrequenzen von Vorteil ist.

Die Erfindung umfasst zudem ein insbesondere elektrisches Gerät, insbesondere ein Körperpflegegerät und/oder ein Körperbehandlungsgerät, beispielsweise einen Rasierapparat, einen Bartschneider, einen Haarschneider, ein Epiliergerät, ein Haarentfernungsgerät, ein Pigmentiergerät, ein Tätowiergerät, ein Stechgerät, eine elektrische Zahnbürste oder dergleichen, wie insbesondere oben erwähnt, mit zumindest einem elektrischen linearen repetitiven Impulsantrieb nach einem der vorhergehenden Ansprüche. Vorteilhaft ist das Gerät als ein Rasierapparat und/oder ein Bartschneider ausgebildet.

### Zeichnungen und Beschreibung des Ausführungsbeispiels

Die Erfindung wird nachfolgend anhand eines Ausführungsbeispiels unter Bezugnahme auf Zeichnungen näher erläutert, hierbei zeigen:
- Fig. 1:: eine schematische Darstellung eines beispielhaften Stechgerätes zur Reiztherapie für organische Haut, welches mit dem erfindungsgemäßen elektrischen linearen repetitiven Impulsantrieb betrieben wird.
- Fig. 2:: Weg-Zeit Diagramme des beispielhaften Stechgerätes betrieben mit dem erfindungsgemäßen Impulsantrieb nach Fig. 1 im Vergleich zu einem Stechgerät mit zeitkontinuierlichem Antrieb nach dem Stand der Technik.

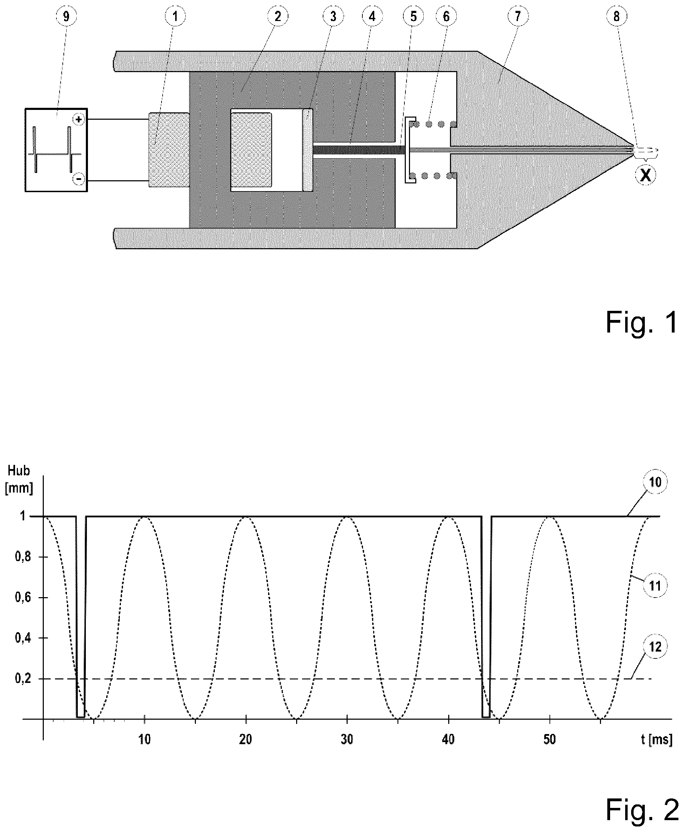

Fig. 1 zeigt beispielhaft ein Stechgerät zur Reiztherapie für organische Haut, betrieben mit dem erfindungsgemäßen elektrischen linearen repetitiven Impulsantrieb, bestehend aus dem Magnetkreis 2 mit Luftspalt 4, der elektrischen Spule 1, dem magnetischen Formgedächtnis-Stellelement 5 mit seinem unmagnetischen Anschlag 3 und einer Rückstelleinheit mit einer Rückstellfeder 6. Insbesondere ist die Rückstelleinheit als die Rückstellfeder 6 ausgebildet. Beim Ansteuern der Spule 1 durch die Steuerelektronik 9 baut sich im Magnetkreis 2 und dem Luftspalt 4 ein Magnetfeld auf, welches das magnetische Formgedächtnis-Stellelement 5 zu einer Dehnung gegen die Feder 6 veranlasst und das Stechelement 8 um einen Betrag X aus dem Gehäuse 7 treibt. Nach Löschung des aufgebauten Magnetfelds durch einen umgekehrt gepolten elektrischen Impuls, bringt die Feder 6 das Stellelement 5 solange in seine Ausgangslage zurück, bis der nächste elektrische Arbeitsimpuls auftritt, so dass das Spektrum an Wiederholfrequenzen vom Stillstand bis zur Grenzfrequenz des Antriebs reicht, wobei jeder Stechvorgang unabhängig von der Wiederholfrequenz die gleiche Energie aufweist. Diese Eigenschaft ist beispielsweise für das beispielhaft genannte Stechgerät zur Reiztherapie von organischer Haut ausschlaggebend, denn die Haut soll in moderaten Weg- und Zeitabständen jeweils nur einmal angestochen werden und das idealerweise immer mit der gleichen Energie pro Stich.

Fig. 2: zeigt Weg-Zeit Diagramme des beispielhaften Stechgerätes betrieben mit dem erfindungsgemäßen Impulsantrieb nach Fig. 1 im Vergleich zu einem Stechgerät mit zeitkontinuierlichem Antrieb nach dem Stand der Technik. Kurve 10 zeigt beispielhaft das zeit diskontinuierliche Weg-Zeit Diagramm der Spitze des Stechelements des beispielhaften Stechgerätes zur Reiztherapie betrieben mit dem erfindungsgemäßen Impulsantrieb mit einer Impulsbreite von 1 ms und einer Einstechtiefe in eine organische Haut von 0,2 mm, gekennzeichnet durch die gestrichelte Linie 12. Im Vergleich dazu zeigt Kurve 10 das zeitkontinuierliche Weg-Zeit Diagramm eines herkömmlichen, dem Stand der Technik entsprechenden Stechgerätes mit einer Frequenz von 100 Hz, also einer Periodendauer von 10 ms. Es ist deutlich erkennbar, dass die Einstechgeschwindigkeit mit dem erfindungsgemäßen Antrieb wesentlich höher ist als mit dem Stechgerät des Stands der Technik. Bei einer beispielsweisen Verringerung der Wiederholfrequenz des erfindungsgemäßen Impulsantriebs und konstant gehaltener Impulsdauer werden die Vorteile des erfindungsgemäßen Impulsantriebs immer größer, was gleichzeitig einer schonenderen Behandlung der Haut entspricht. Bei einem Vergleich beider Systeme ist zudem sehr deutlich ersichtlich, dass der erfindungsgemäße Impulsantrieb sowohl in Bezug auf den Leistungsverbrauch, den Wirkungsgrad bezüglich der Einstecharbeit, die System-Verlustleistung, die Vibrationen und die Betriebsdauer mit Akkus/Batterien wesentliche Vorteile aufweist.

Die in vorstehender Beschreibung, der Zeichnung und den Ansprüchen offenbarten Merkmale der Erfindung können die Verwirklichung der Erfindung sowohl einzeln als auch in beliebiger Kombination in ihren verschiedenen Ausführungsformen bilden, unabhängig von der Zusammenfassung, in einzelnen Ansprüchen oder deren Rückbeziehung.

Insbesondere ist, wie oben erwähnt, ein anderes Gerät wie beispielsweise ein Körperpflegegerät und/oder ein Körperbehandlungsgerät mit einem linearen repetitiven Impulsantrieb denkbar. Insbesondere ist ein analog zu dem beschriebenen Stechgerät ausgebildeter Rasierapparat und/oder Haarschneider und/oder Bartschneider denkbar.

## Patentansprüche

1. Körperpflegegerät und/oder Körperbehandlungsgerät, insbesondere Rasierapparat, Bartschneider, Haarschneider, Epiliergerät, Haarentfernungsgerät, Pigmentiergerät, Tätowiergerät, Stechgerät, elektrische Zahnbürste oder dergleichen, mit zumindest einem elektrischen linearen repetitiven Impulsantrieb mit
- einem Magnetkreis (2) mit Luftspalt (4) und einer elektrischen Spule (1),
- einem magnetischen Formgedächtnis-Stellelement (5), welches in dem Luftspalt (4) des Magnetkreises (2) angeordnet ist,
- einer Rückstelleinheit (6)
- und einer Steuerungselektronik (9), wobei
der elektrische lineare repetitive Impulsantrieb seine Energie zeitdiskontinuierlich, in Impulsen gebündelt mit einer Wiederholfrequenz abgibt, wobei die Wiederholfrequenz des elektrischen linearen repetitiven Impulsantriebs werksseitig auf den jeweiligen Anwendungsfall oder manuell vom Anwender zur Optimierung der jeweiligen, auch individuellen Anwendung einstellbar ist, und wobei bei einem Verändern der Wiederholfrequenz die abgegebene Energie pro Impuls konstant ist.

2. Körperpflegegerät und/oder Körperbehandlungsgerät nach Anspruch 1, **dadurch gekennzeichnet, dass** zur Minimierung der Rückstellzeit des Impulsantriebs ein umgekehrt gepolter Stromimpuls, also ein Löschimpuls, welcher im Umkehrpunkt des Arbeitsimpulses ausgelöst wird, das im Luftspalt (4) des Magnetkreises (2) befindliche magnetische Formgedächtnis-Stellelement (5) zu einer Dehnung veranlasst, wobei dieser Stromimpuls das Magnetfeld des magnetischen Kreises schlagartig löscht und dadurch das Stellelement ungehindert und vorzugsweise mit maximal möglicher Schnelligkeit durch die Rückstellfeder in seine Ausgangslänge zurückgebracht wird.

3. Körperpflegegerät und/oder Körperbehandlungsgerät nach Anspruch 1 oder 2, **dadurch gekennzeichnet, dass** zur Minimierung der Anstiegszeit des Impulsantriebs, die Rückstelleinheit frei von einer Rückstellfeder ausgebildet ist und ein weiteres gesteuertes magnetisches Formgedächtnis-Stellelement umfasst und damit die Anstiegszeit durch Entfall der hemmenden Wirkung der Rückstellfeder weiter verbessert ist.

4. Körperpflegegerät und/oder Körperbehandlungsgerät nach einem der vorhergehenden Ansprüche, **dadurch gekennzeichnet, dass** die Geräuschentwicklung des Festkörper-Stellelements (5) des Impulsantriebs durch geräuschminimierende Anstiegs- und Abfallkurvenverläufe des Magnetfelds verbessert ist, wobei das Gerät ohne eine leistungsmindernde akustische Dämpfung der Kinematik und ohne ein den Impulsantrieb umgebendes Gehäuse ausgebildet ist.

## Claims

1. Body care apparatus and/or body treatment apparatus, in particular a shaver apparatus, a beard cutter, a haircutter, an epilator apparatus, a hair-removal apparatus, a pigmenting apparatus, a tattoo apparatus, a pricking apparatus, an electric toothbrush, or the like, with at least one electric linear repetitive pulsed drive comprising
- a magnetic circuit (2) with an air gap (4) and an electric coil (1),
- a magnetic shape-memory adjustment element (5) which is arranged in the air gap (4) of the magnetic circuit (2),
- a reset unit (6)
- and a control electronics unit (9), wherein
the electric linear repetitive pulsed drive emits its energy in discrete-time fashion, bundled in pulses with a repetition frequency, wherein the repetition frequency of the electric linear repetitive pulsed drive is adjustable for the respective application during manufacture or is manually adjustable by the user to optimize the respective, also individual, application and wherein if the repetition frequency is changed, the energy emitted per pulse remains constant.

2. Body care apparatus and/or body treatment apparatus according to claim 1, **characterized in that** in order to minimize the reset time of the pulsed drive an inversely polarized current pulse, i.e. a deletion pulse which is initiated at the reversal point of the working pulse, causes an expansion of the magnetic shape-memory adjustment element (5) which is situated in the air gap (4) of the magnetic circuit (2), wherein this current pulse abruptly deletes the magnetic field of the magnetic circuit and thus the adjustment element is returned to its original length by the reset spring without hindrance and preferably with the maximum possible speed.

3. Body care apparatus and/or body treatment apparatus according to claim 1 or 2, **characterized in that** in order to minimize the rise time of the pulsed drive, the reset unit is embodied free of a reset spring and comprises a further controlled magnetic shape-memory adjustment element and thus the rise time is further improved by dispensing with the inhibiting effect of the reset spring.

4. Body care apparatus and/or body treatment apparatus according to one of the preceding claims, **characterized in that** the noise production of the solid-state adjustment element (5) of the pulsed drive is improved by noise-minimizing rise and fall curves of the magnetic field, wherein the apparatus is embodied without a performance-reducing acoustic damping of the kinematics and without a housing surrounding the pulsed drive.

## Revendications

1. Appareil de soin corporel et/ou appareil de traitement corporel, en particulier un appareil de rasage, un coupe-barbe, un coupe-cheveux, un appareil éplilateur, un appareil d'épilation, un appareil de pigmentation, un appareil de tatouage, un appareil de piquage, une brosse à dents électrique, ou similaire, avec au moins un entraînement électrique linéaire à impulsions répétitives avec
- un circuit magnétique (2) avec une fente d'air (4) et une bobine électrique (1),
- un élément régleur magnétique à mémoire de forme (5) qui est disposé dans la fente d'air (4) du circuit magnétique (2),
- une unité de rappel (6)
- et une électronique de commande (9), où
l'entraînement électrique linéaire à impulsions répétitives émet son énergie de manière discontinue dans le temps, groupée par impulsions avec une fréquence de répétition, où la fréquence de répétition de l'entraînement électrique linéaire à impulsions répétitives est réglable pour le cas d'application respectif pendant la fabrication ou est réglable manuellement par un utilisateur pour optimiser l'application respective, également individuelle, et où
dans une modification de la fréquence de répétition l'énergie émise par impulsion reste constante.

2. Appareil de soin corporel et/ou appareil de traitement corporel selon la revendication 1, **caractérisé en ce que** pour minimiser le temps de rappel de l'entraînement à impulsions, une impulsion de courant inversement polarisée, c'est-à-dire une impulsion de suppression, qui est initiée au point d'inversion de l'impulsion de travail, provoque une dilatation de l'élément régleur magnétique à mémoire de forme (5) qui se trouve dans la fente d'air (4) du circuit magnétique (2) à, où cette impulsion de courant éteint brusquement le champ magnétique du circuit magnétique et ainsi l'élément régleur est ramené à sa longueur d'origine sans entrave et de préférence avec la vitesse maximale possible par le ressort de rappel.

3. Appareil de soin corporel et/ou appareil de traitement corporel selon la revendication 1 ou 2, **caractérisé en ce que** pour minimiser le temps de montée de l'entraînement à impulsions, l'unité de rappel est formée libre d'un ressort de rappel et comporte un élément régleur magnétique à mémoire de forme contrôlé de plus, le temps de montée étant encore amélioré à cause de la suppression de l'effet inhibiteur du ressort de rappel.

4. Appareil de soin corporel et/ou appareil de traitement corporel selon l'une quelconque des revendications précédentes, **caractérisé en ce que** la production de bruit de l'élément régleur à corps solide (5) de l'entraînement à impulsions est amélioré par des courbes de montée et de descente minimisant le bruit du champ magnétique, où l'appareil est formé sans un amortissement acoustique de la cinématique réduisant une performance et sans un boîtier entourant l'entraînement à impulsions.
